# EUROPEAN PATENT SPECIFICATION

(11) **EP 4 221 813 B1**
(45) Date of publication and mention of the grant of the patent: **03.04.2024**
(21) Application number: 21790093.5
(22) Date of filing: 28.09.2021
(51) Int. Cl.: A61N 1/04

(54) **A SURFACE ELECTRODE**
OBERFLÄCHENELEKTRODE
ÉLECTRODE DE SURFACE

(30) Priority: 29.09.2020 EP 20461562
(43) Date of publication of application: 09.08.2023
(73) Proprietor: Neuro Device Group S.A., 01-510 Warsaw (PL)
(72) Inventor: GIERGIELEWICZ, Mariusz, 04-501 Warszawa (PL); PROKOPCZYK, Pawel, 04-501 Warszawa (PL); JANKIEWICZ, Adam, 04-501 Warszawa (PL); MALEJ, Krzysztof, 04-501 Warszawa (PL)
(74) Representative: Kancelaria Eupatent.pl Sp. z.o.o
(86) International application number: PCT/EP2021/076628
(87) International publication number: WO 2022/069464

(56) References cited:
- WO-A1-2016/077236
- ES-T3- 2 618 838
- US-A1- 2014 213 875
- US-A1- 2014 249 613
- US-A1- 2019 374 766

## Description

### TECHNICAL FIELD

The present invention relates to a surface electrode for non-invasive electrostimulation including electrostimulation of desired body parts, including, head, arms, legs, bag, organs, tissues and/or tissues cells, e.g. brain, nerve system, nerve cells, muscles, skin, epidermis etc.

### BACKGROUND

A surface electrode is a device connectable to a stimulation unit which constitutes an electricity source. During the electrostimulation, the surface electrode stays on a skin external surface, playing an important role in interfacing the skin tissue with the stimulation unit to which it is connected. The surface electrode may be attached to the skin, e.g., the electrode for use on head, may be attached using cap or headbands. When current, typically consisting of a series of electrical waveforms, is applied to the surface electrode, placed on the skin substantially overlaying the nerve structures, an electric field is generated between two electrodes (anode and cathode) and ions which creates a current in the desired place of the body part or tissue.

In other words, the surface electrode constitutes a terminal through which electrical current passes into the underlying tissue. At the electrode-tissue interface a conversion occurs between the current of electrons (driven from the stimulation unit through the electrode) and the current of ions in the tissue.

Engineering of the surface electrodes aims at proper user's cutaneous sensation during the electrostimulation, including the absence of skin burns and minimized skin irritation, along with a proper electro-stimulation of the target element(s) of the body as the design of the surface electrode shapes the current flow through the target element(s) of the body.

Depending on the given treatment requirements, the surface electrodes are optimized for a low impedance. An impedance that is too high leads to elevated voltage resulting in increased irritation of the skin followed by skin burns.

The surface electrodes can be of various shapes, wherein typical shapes include snap, pin, pellet, disk, sheet, or mesh, with the electrode body made of metal or conductive rubber. The shape of the electrode can be adjusted to application. Generally the smaller the electrode size, the higher the current density. Thus, the electrode size is a factor limiting the maximum amplitude of stimulation current/voltage.

An important aspect of the electrostimulation with the surface electrode is the conductive medium used at the electrode-tissue interface. The preferred conductive medium is an electrolyte, typically in a liquid, semi-liquid or solid form, such as saline, conductive paste, or conductive gel. The conductive medium forms a conductive layer between the user's skin and the electrode body providing therein a conversion of electrons from the electrode into appropriate ions. The conductive layer should be present during the whole period of the electrostimulation to avoid skin irritation and burns. However, this is difficult to achieve, as the conductive medium can evaporate during the duration of the electrostimulation, causing discontinuities in the conductive layer. An assembly of the surface electrode typically comprises holding means for holding the conductive medium in a form of a continuous conductive layer. The holding means typically include sponge materials soaked with the conductive medium. Nonetheless, the latter require periodic feeding with a fresh dose of saline dose, due to the evaporation of saline, which imposes various technical difficulties with feeding the sponges with saline.

Further, there exist self-adhesive electrodes, comprising the conductive medium, a gel or a hydrogel formed into the conductive layer to cover the electrode surface. This conductive layer is rigid and may be attached directly, or with the aid of an adhesive, to the electrode surface.

Yet further, there exist dry electrodes that do not use external conductive medium. Instead, the dry electrodes use sweat from the user's skin. These electrodes are optimized for work with high impedance. However, high-impedance solutions may work reasonably well in biosignal recording, while in case of electrostimulation the use of current flow between high-impedance electrodes leads to elevated voltage resulting in increased irritation of the skin followed by skin burns. Impedance of dry electrodes is especially high shortly after positioning of electrodes on the skin, thus limiting possibility to even start electrostimulation. After some time impedance gets better due to sweating, wherein sweat may act as conductive medium.

Good quality of the surface electrode operation mode is further accomplished by providing a substantially large contact surface between the electrode, the conductive medium and the skin. However, this is difficult to achieve due to the electrode shape, which typically does not correspond with uneven body shape, and may be even further diminished at hairy skin regions, such as scalp regions, e.g. during brain electrostimulation, due to the presence of hair which significantly reduce the above-mentioned contact surface.

The above aspects of surface electrode operation, i.e. the required large contact surface (electrode / conductive medium / skin) along with a proper application of the conductive medium onto the skin and long-lasting maintenance of the formed conductive layer, most preferably during the whole electrostimulation, are important factors which, if properly combined, can ensure good operational mode of the surface electrode, including minimized skin irritations together with selected impedance regime in the required application time.

Among the known architectures of the surface electrodes, there are known transcranial stimulation electrodes for scalp application and brain electrostimulation, especially neuromodulation of patients with brain injuries as well as electrostimulation of various psychiatric conditions like major depressive disorder. During the electrostimulation, the electrode stays in electrical contact with the stimulation unit, i.e. a current or voltage generator that generates stimulation current or voltage substantially transdermally delivered via the surface electrode, and further via the layer of conductive medium to the tissue. Since the conductive medium requires even distribution over the scalp hair, this transcranial electrode can be either equipped with a saline-soaked sponge, or it can be provided with an electroconductive paste to form the conductive layer of conductive medium. The proper formation of the conductive layer of conductive medium enables the electrode to achieve a low impedance operational mode. Nonetheless, the process for preparing the desired conductive layer of conductive medium is complex, time-consuming, as well as it requires the engagement of highly-qualified and experienced medical personnel. Therefore, the preparation and installation of the electrode can be carried out only at points of care, e.g. hospitals.

Proper positioning of the surface electrode on hairy skin regions, especially in the presence of dense and/or long hair is very difficult. Hair may form an additional undesired layer between skin and electrode, and further affects the formation and maintenance of the continuous conductive layer of conductive medium, during the electrostimulation. Typically, flat electrodes are difficult to apply in this condition. Alternatively there were proposed new designs of electrodes engineered as electrode units of the plurality of components or component electrodes usually of pin-like or spike-like shapes, attached to a common substrate forming a brush- or comb-like design. This design facilitates application, as the plurality of component electrodes can be positioned between hairs.

Nonetheless, the brush-like or comb-like design of the surface electrode encounters various problems associated mainly with the application of the conductive medium into the area of the skin-electrode interface, and further maintenance of the conductive layer during the whole electrostimulation to provide proper transfer of the electrical charges and to avoid skin irritation and burns. Furthermore, due to limited contact area of pins or spikes, a use of component electrodes can lead to undesirably increased impedance and excessive current density resulting in low simulation comfort and/or skin injuries. This drawback may be further amplified by uneven contact of the electrodes with the skin, as the units of component electrodes attached to the common substrate can be rigid, unfitting the body shape.

From the patent literature, there are known various designs of the surface electrodes for electrostimulation.

A European patent publication EP3142744 describes a transcranial stimulation electrode that consists of a super-porous hydrophilic material having a first relaxed state and a second expanded state. In the latter state, the super-porous hydrophilic material is breakable to at least partly enclose at least one hair. The current is driven from the electrode surface, through the hydrophilic material, to the hairy skin. The super-porous hydrophilic material of the electrode comprises polymerized acrylamide, with additives such as: polymerized acrylic acid, polymerized sulfopropylacrylate potassium salt, polymerized methylene bisacrylamide.

US patent application us2014213875 describes an electrode device for use on skin surfaces - for a defibrillating shock to be applied to a patient through the amount of the electrically conductive gel. The device consists of at least one electrode unit having a plurality of protrusions, e.g. rod-shaped protrusions made from a conductive material, serving as component electrodes of the electrode unit. Each of the electrode units is supplied with a conducive gel delivered, via a conduit, from a reservoir arranged at a certain distance from the electrode unit. Each of the protrusions has a constant thickness along its entire length.

An international patent application WO2016077236 describes an electrode device in a form of garment for skin applications. The device comprises an electrode attached to the garment, via its first surface. A second surface of the electrode is to be placed in contact with a portion of a user's skin when the garment is worn. The electrode takes a flat form of an electro-conductive layer, to adhere to the skin.

Spanish patent publication ES2618838 describes a device for transdermal application of active substances to a patient. The device can perform small openings (or micro-pores) within a patient's skin. The device consists of a disposable substrate, a conduit extending between an upper and lower surface of the substrate, a duct connected to the conduit, an antiadhesive lining, and removable adhesive tape. This device does not comprise electrodes for providing current flow and, thereby, it falls in the technical field removed from the topics related to the surface electrodes for non-invasive electrostimulation.

US patent application US2019/374766 describes a system for providing electrical stimulations to users. The system consists of an electrode unit having an array of component electrodes having substantially uniform thickness along their lengths. Each component electrode is a permeable body serving as wet electrode contact that comprises a fluid absorbing material configured to provide an electrically conductive connection to a power source of the system. Optionally, the component electrodes may comprise deformable elements, e.g. in a form of a flexible sponge, hydrogel, or a polymer with shape-memory. These elements are supposed to maintain the desired amount of conductive medium close to the user's skin.

US patent application US2014249613 describes a medical device built from a plurality of therapy electrodes that are electrically coupled to a control unit via connection means. The electrodes are capable of delivering therapeutic defibrillating shocks to a patient. The electrodes are made from conductive stitching that is incorporated in a fabric. Thus, the device does not comprise electrode-like components that protrude from the substrate so as to provide distal and proximal ends of the electrodes.

Taking into account the above drawbacks, the present invention aims to develop a surface electrode of an improved contact area: electrode / conductive layer of conductive medium / skin, as well as facilitated formation and maintenance of the conductive layer of a conductive medium during the whole electrostimulation period, either at the beginning of the treatment, as well as if prolonged. Furthermore, the present invention aims to provide the surface electrode of simplified handling enabling the user to utilize the surface electrode at home conditions, e.g. only under medical supervision or purely on medical instruction, depending on the individual considerations.

### SUMMARY

There is disclosed a surface electrode for use on a skin according to the appended claims.

### BRIEF DESCRIPTION OF THE DRAWINGS

These and other objects of the invention presented herein are accomplished by providing a surface electrode as described herein. Further details of the electrode, its technical features and various advantages will become more apparent from the following detailed description of the preferred embodiments shown in a drawing, in which:
Figs. 1A - 1C present an electrode unit of a surface electrode according to the present invention;
Figs 1D - 1G presents a substrate of the electrode unit with component electrodes arranged therein;
Fig. 2 presents a system for supplying a conductive medium of the electrode unit of the surface electrode according to the present invention;
Fig. 3 presents an embodiment of the surface electrode according to the present invention;
Fig. 4 schematically presents a working principle of the surface electrode according to the present invention;
Fig. 5 presents exemplary fastening means of the electrode unit of the surface electrode according to the present invention, as well as external conductive medium reservoir;
Figs 6A, 6B present two embodiments of the component electrodes of the surface electrode according to the present invention.

### DETAILED DESCRIPTION

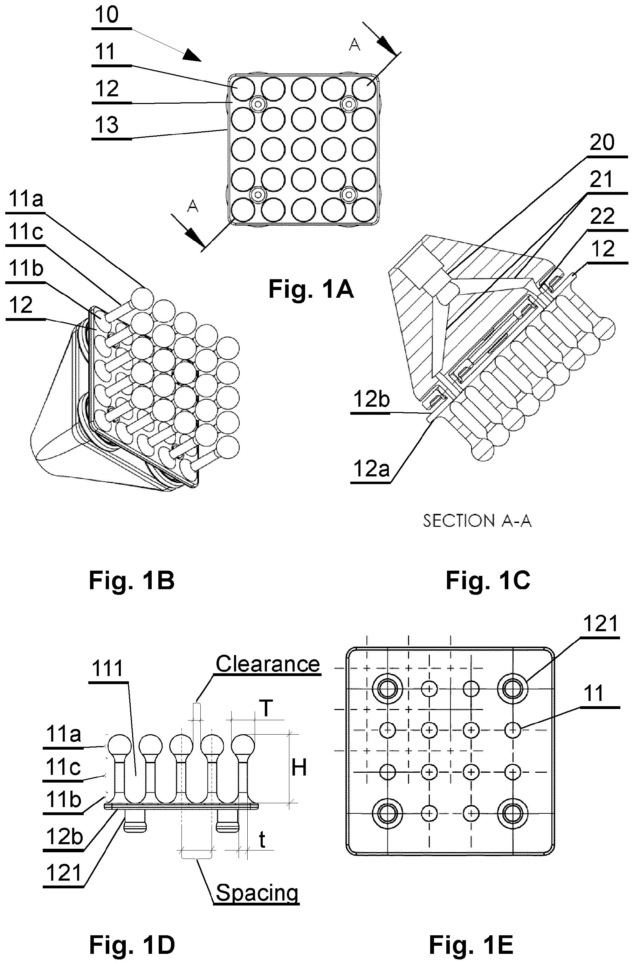

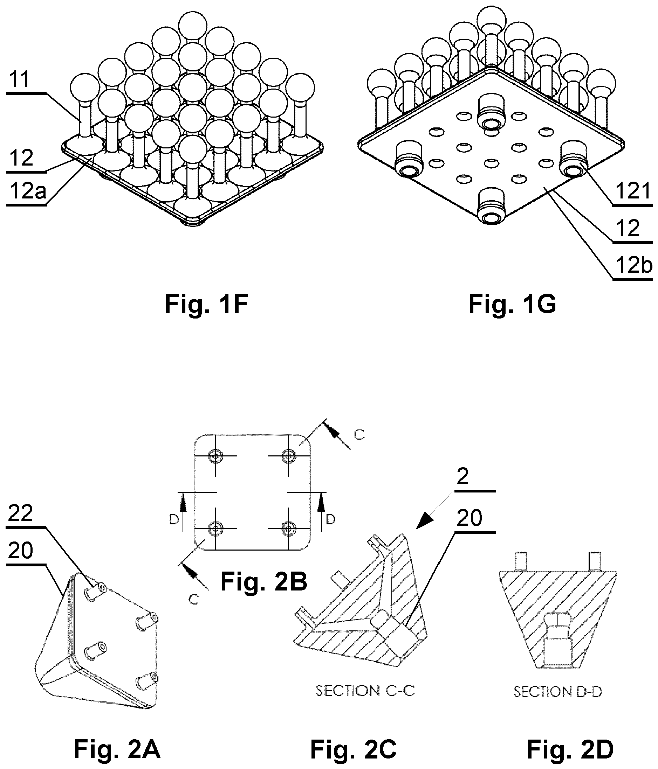

The surface electrode comprises at least one electrode unit 10, and more preferably a plurality electrode units 10. Each of the electrode units 10 comprises a plurality of component electrodes 11, preferably each of the same length, and disposed on a common substrate 12, as shown in Fig 1. The substrate 12 comprises a front side 12a on which the component electrodes 11 are arranged 11, and a back side 12b.

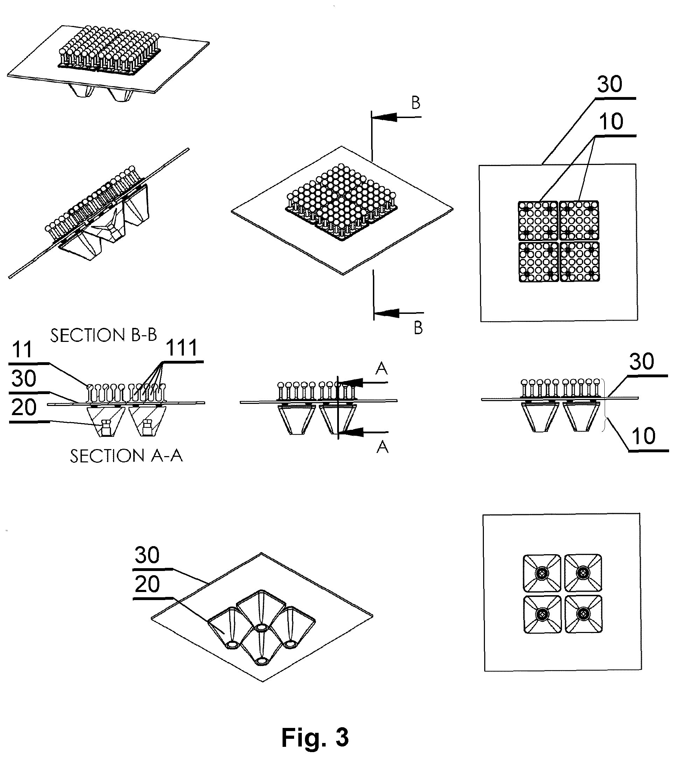

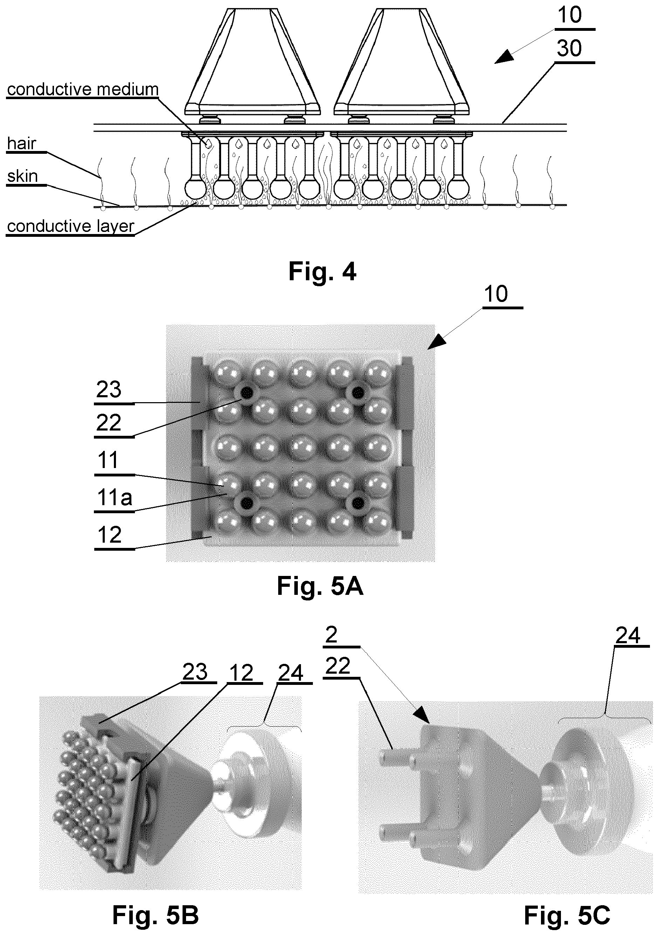

The surface electrode may comprise a plurality electrode units 10, e.g. two electrode units 10, three electrode units 10, four electrode units 10, or more than four electrode units 10, each unit 10 comprising a plurality of component electrodes 11, as shown in Fig. 3 and 4.

The component electrodes 11 of each electrode unit 10 protrude from the substrate 12 in the same direction, thereby forming a brush-like structure. Each component electrode 11 comprises a proximal end 11b connected with the substrate 12, a distal end 11a protruding outside the substrate 12, and a middle portion 11c between the distal end 11a and the proximal end 11b of the component electrode 11. The distal ends 11a together determine a working area of the surface electrode to be applied on the skin, at the desired treatment zone.

The layout of the component electrodes 11, within a single electrode unit 10, may take various forms, for example, a form of one or more than one row of component electrodes 11 further arranged in series or in parallel, as shown in Fig 1, in which the component electrodes 11 together form a square layout of sides: 5x5 component electrodes 11.

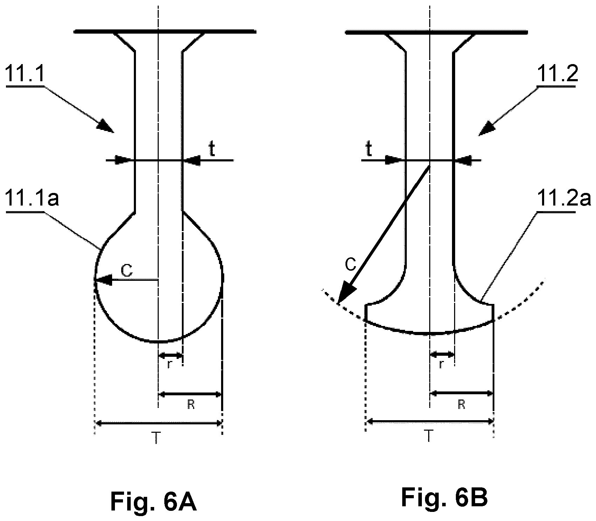

The distal ends 11a of the component electrodes 11 may be of various shapes, preferably selected from the group consisting of sphere 11.1a, hemisphere, roll, cylinder, stick-like, pin-like, spike-like, mushroom-like shape (spherical-like segment) 11.2a and a pyramidal shape, as schematically shown in Figs 6A, 6B. The proximal ends 11b of the component electrodes 11 may be of various shapes, preferably selected from the group consisting of roll, cylinder, stick-like, pin-like, spike-like and pyramidal shape. The middle portions 11c of the component electrodes 11, 11.1, 11.2 may be in a shape of a solid of revolution, e.g. in a cylindrical shape.

Within a single component electrode 11, the thickness (T) of its distal end 11a is greater than the thickness (t) of its middle portion 11c, as schematically shown in Fig. 1D and Figs 6A, 6B. The thickness (T, t) may be expressed, for example, by size or radius of the distal end 11a, 11.1a, 11.2a, and the middle portion 11c, respectively. Thus, for example, for the spherical, circular, cylindrical or mushroom-like shape of the distal end 11a, 11.1a, 11.2a, the radius (R) of the distal end 11a, 11.1a, 11.2a of single component electrode 11 may be greater than the radius (r) of its middle portion 11c, as schematically shown in Fig. 6A, where the component electrode 11.1 has the distal end 11.1a in the shape of a sphere, and in Fig. 6B where the component electrode 11.2 has the distal end 11.2a in the mushroom-like shape. Preferably, the radius (R) of the distal end (11a) is 2 to 5 times greater than the radius (r) of the middle portion 11c within the single component electrode 11, e.g. the radius (R) of the distal end 11a may be 1.5 mm and radius of the middle portion 11c may be 0.5 mm. The thickness (T) of the distal end 11a of the component electrode 11 (within the mentioned range) is small enough for the surface electrode to be inserted through the hair and large enough to provide reasonable skin-electrode contact between the hair. The larger area of a surface of the distal end 11a, e.g. having the thickness (T) > 5mm, upon contact with the skin - may lead to gathering hair underneath the component electrode leading to an increase in impedance. Also, using the component electrodes of a smaller area of surface of the distal end 11, e.g. having the thickness (T) <0.8 mm, upon contact with the skin, may lead to an increase in impedance - due to low contact area, and/or may cause discomfort due to increased pressure on the skin.

The thickness (t) of the middle portion 11c, which is smaller than the thickness (T) of the distal end 11a, 11.1a, 11.2a brings larger space where hair can be allocated, notwithstanding large contact area: electrode / conductive medium / skin provided by the distal ends 11a, 11.1a, 11.2a. Thus, adequately greater thickness (T) of distal ends 11a, 11.1a, 11.2a leads to a larger working area of the surface electrode, while smaller thickness (t) of middle portions 11c provides a respective free space volume 111 formed between the component electrodes 11 for hair, i.e. the volume where hair may be allocated during positioning the surface electrode on the hairy skin regions. To this end, the hair is gathered between the middle portions 11c and over laterally extending (i.e. wider) distal ends 11a, 11.1a, 11.2a of the component electrodes 11, 11.1, 11.2. Thereby, the hair does not cause increased impedance, during the operation of the surface electrode.

Preferably, the distal ends 11a, 11.1a, 11.2a of the component electrodes 11, 11.1, 11.2 have convex surfaces for contacting the skin, wherein said convex surfaces are defined by a radius of curvature (C) of said convex surfaces (Figs 6A, 6B). Further, for the distal ends 11a of convex surface shape such as the shape of sphere 11.1a, hemisphere, or mushroom-like shape 11.2a, the greater the radius of curvature (C) of the convex surface of the distal end, the bigger the contact area of electrode/conductive medium /skin.

In preferred embodiments of the component electrodes 11.2 having the mushroom-like shape of the distal ends 11.2a (Fig. 6B), the radius of curvature (C) may be at least 3 mm or more, where the radius (R) of the distal end 11.2a equals 1.5 mm and radius (r) of middle portion 11c equals 0.5 mm. Such the dimensions of the radius of curvature (C) enable all the components electrodes 11.2, and thereby the electrode units 10, to achieve improved contact area, especially at the hairy skin regions, such as scalp. Namely, in the case of mushroom-like shape of the distal ends, the radius of curvature (C) is big enough, compared to the radius R of the distal end 11.2a, wherein the free space volume 111 is maintained substantially large, and the maintained certain curvature (that arises from C) at the contact area electrode/skin enables one to take-up the hair in an undisturbed manner, during electrode positioning. In addition to the above, the mushroom-like distal ends 11.2a are substantially short, compared to these of sphere shape 11.1a for which the R value equals the C value. This makes the whole electrode unit 10 more hair loadable, and the introduction of the hair into the free space volume 111, between distal ends 11.2a of the adjacent component electrodes 11 is facilitated.

Furthermore, the shape of the components electrodes 11 having the thickness (T) of its distal ends 11a, 11.1a, 11.2a which is greater than the thickness (t) of the middle portions 11c provides a relatively denser arrangement of the component electrodes 11 on the surface, as the contact area with the skin is improved, whilst maintaining the free space volume 111 for hair substantially large. This provides lower impedance and more regular distribution of the current density within the area of the skin that is covered by the surface electrode (lower current density at the contact area of each contact electrode 11). This, in turn, provides improved comfort and safety in the use of the surface electrode according to the present disclosure. The user does not feel the current (or feels it only at a negligible level), due to the achieved lower voltage resulting from lower impedance, and lower current density in the respective contact areas: electrode/skin. This further reduces or even eliminates the risk of skin burns.
For this reason the enlarged distal ends 11a. 11.1a, 11.2a of various shapes provide further improvement in the operational behavior of the surface electrode.

Moreover, the developed construction of the surface electrode allows for increased free space volume 111 for hair, thereby, the adjacent distal ends 11a, 11.1a, 11.2a may be close to the other, providing certain clearances one from another, as shown e.g. in Fig 1D. For example distal ends 11a, 11.1a, 11.2a may be spaced from each other by the clearance of 1 to 5 mm. Such small size of the clearances between the adjacent distal ends 11a, 11.1a, 11.2a allows for an increase in density of the component electrodes 11, 11.1, 11.2 per area unit, depending on special needs. Thus the component electrodes density may be designed more flexibly, taking into account that the greater the contact area: electrode / conductive medium / skin, the lower the contact impedance and lower the current density in the skin, enhancing the comfort and safety of the user. Due to the developed distal ends 11a, 11.1a, 11.2a as an integral part of the component electrodes 11 - made of the material of the component electrode 11, the size of the clearances remains unchanged. In other words, the clearances are of the same size, before, during, and after the electrostimulation of the user.

As shown in Fig. 1D and 3, the free space volume 111 is restricted by the substrate 12 from one side and the distal ends 11a of the component electrodes 11 from another side. For example, the distal end 11a may have length of 3 mm and the middle portion 11c may have length of 6 mm. Alternatively, the length of middle portion may be increased to better suit users with longer and/or thicker hair. The length of distal end 11a may vary depending on its shape. Additionally, the radius (R) of distal ends may be related to density of component electrodes 11 in electrode unit, i.e. the radius (R) of distal end may be adjusted to provide specific spacing between two adjacent component electrodes 11, and thereby specific clearance, e.g. the clearance of 1 mm. Different spacing values are possible, however, lower values may prevent the hair to move into free volume space 111, while higher values may lead to a decrease of total contact area due to reduced number of component electrodes 11 per area unit. Preferably, a higher number of the components electrodes 11, 11.1, 11.2 is required to provide a total simulation surface sufficient to meet limits of maximum current per surface, which can be for example 25.45 A/m². The array of the component electrodes 11, 11.1, 11.2 comprising a plurality of the components electrodes, is used to extend the total contact area: electrode/conductive medium /skin.

Furthermore, the material body of the component electrode is non-expandable e.g. upon contact with moisture, e.g. derived from the conductive medium or swept. Thereby, the clearances, provided by the spacings between the respective distal ends 11a, 11.1a, 11.2a of the component electrodes, stay unchanged during the whole electrotreatment, which assures controlled conditions on the electrostimulation. Also, after the electrostimulation is finished, the unchanged clearances (and thereby unchanged spacings) help with removing the surface electrode without hair damage, thus providing yet further enhanced comfort to the user.

The component electrodes 11 may be made of various suitable non-expandable materials, preferably electroconductive and biocompatible with the user's body. For example, the component electrodes 11 may be made of stainless steel, for instance, 316L stainless steel which provides good resistance to degradation processes and relatively low impedance. Another suitable example of the material for the component electrodes 11 is gold or silver coated with silver chloride. Alternatively, electroconductive polymers may be used such as, e.g., ethylene propylene diene monomer (EPDM) matrix mixed with carbon or stainless steel fibers.

Each electrode unit 10 is connectable, via connectors (not shown in the drawing for clarity) such as cables, to an output of a stimulation unit constituting a power supply which can be a voltage supply and/or current supply, depending on how the electrode units are to be powered so as to provide the electrical connection with each component electrode 11. This can be realized with various suitable means, such as used in the known surface electrode structures. Nonetheless, according to the present invention, each electrode unit 10 may be provided with an independent connector, thus, providing an independent electro-stimulation mode of each electrode unit 10. For example, during the performed electrostimulation, only selected electrode units 10 may be turned on, or provided with a different stimulation mode than the other electrode units 10, thereby simplifying adaptation of the working area of the surface electrode and adjusting the stimulation current-voltage of each electrode unit 10 with respect to the special user's needs.

Each electrode unit 10 further comprises a system 2 for supplying a conductive medium to the component electrodes 11 for forming a conductive layer between the distal ends 11a of the component electrodes 11 and skin, as shown schematically in Fig. 4. The system 2 for supplying a conductive medium of one electrode unit 10 may work independently from those of the other electrode units 10, or the systems for supplying a conductive medium of all the electrode units 10 of the surface electrode may work in co-operation, depending on the electrostimulation needs. The system 2 for supplying a conductive medium of each electrode unit 10 comprises a manifold 20, arranged on the back side 12b of the substrate, i.e. on the substrate side 12b that is opposite to the front side 12a with the protruding component electrodes 11. The manifold 20 is arranged opposite to the working area of the surface electrode so as to not disturb the operational condition of the surface electrode. The manifold 20 comprises ducts 21, each ended with the outlet tube 22 embedded through the substrate 12 material, across the substrate 12. Each outlet tube 22 delivers the conductive medium from the manifold 20, to the working area of the surface electrode, thus forming the conductive layer of the conductive medium, as shown in Fig. 4.

The conductive medium, preferably in a liquid state, may be fed to the manifold 20 from the conductive medium reservoir 24. The feeding may be accomplished by the use of any suitable means, such as a pump, pumping the conductive medium from the reservoir 24 to the manifold 20. In one embodiment of the working mode of the surface electrode, the conductive medium is delivered from the conductive medium reservoir to the component electrodes 11 only once per electrostimulation session, at the beginning of electrostimulation. In another embodiment of the working mode of the surface electrode, the conductive medium is delivered form the medium reservoir to component electrodes 11 through entire electrostimulation session, with predetermined dosage regime. The flow rate of the conductive medium, and thus the amount of the conductive medium dosed to the working area of the surface electrode, may be regulated by using various means, such as desired pump settings. The conductive medium may be preferably in a liquid state, e.g. the saline solution may be used, which has similar physical characteristics to body fluids, such as sweat. Furthermore, the system 2 for supplying a conductive medium may be configured for feeding the working area with less dense conductive medium, e.g. in form of aerosol, such as mixture of saline and air. Alternatively, the system may supply conductive medium e.g. in a gel state or similar.

Each electrode unit 10 may be provided with several outlet tubes 22, preferably evenly spaced across the substrate 12, as shown in Fig. 1A so as to deliver an equal amount of the conductive medium over the whole skin area that undergoes electrostimulation, thus, forming a continuous conductive layer. For example, one electrode unit 10 may comprise four outlet tubes 10, or more than four outlet tubes 10. In the case of very dense hair, the outlet tube 22 may be arranged between every two component electrodes 11 within the electrode unit 10. Preferably, the conductive medium is dosed dropwise, periodically or continuously, during the electrostimulation, depending on the needs. In some embodiments the conductive medium may be supplied under elevated pressure, to effectively and evenly spray the component electrodes 11, hair and the user's skin which is arranged under the surface electrode, during the electrostimulation.

The present invention is especially suitable for hairy skin regions. During the electrostimulation, the hair is disposed in the free space volume 111 between the component electrodes 11, i.e. over the distal ends 11a and between the middle portions 11c. The conductive medium is dosed dropwise from the outlet tubes 22 (not shown in Fig. 4 for clarity). The conductive medium forms the conductive layer between the distal ends 11a of the component electrodes 11 and the skin. The treatment may be carried out for the prolonged time, as the evaporating conductive medium is substituted with its new (fresh) portion, in the continuous or batch mode, depending on the need, thereby, keeping the proper thickness of the conductive layer during the whole treatment. Alternatively, during prolonged electrostimulation sessions, the conductive medium may be naturally substituted by the user's sweat, thus new portion of conductive medium may not be necessary. Impedance of the electrode-skin contact may be measured by stimulation unit to detect whether it is necessary to supply conductive medium during electrostimulation.

Furthermore, the surface electrode provides an increased contact area: electrode / conductive layer of conductive medium / skin as the developed construction enables the surface electrode to fit the body shape, e.g. head. This is achieved due to the presence of the electrode units 10 that are connected to each other via a deformable layer 30 of material. Alternatively, electrode units 10 may be attached to a substrate, wherein multiple substrates may be deformably connected to each other using mechanical joints.

In details, according to the present invention, the electrode units 10 are attached to the deformable layer 30 of material, such as fabric, foil, or flexible and/or ductile sheet of material. Further, the material constituting the deformable layer 30 may be a part of a cap or band, in particular of a headcap or a headband, serving to stabilize the surface electrode on the body part. The attachment of each electrode unit 10 to the deformable layer 30 is provided by sandwiching the deformable layer 30 by the substrate 12 of the electrode unit 10 from one side, and by the manifold 20 from another side of the deformable layer 30, as schematically shown in Fig. 3 and 4. The attachment may be accomplished for example by snap fasteners comprising a pair of interlocking parts, a male part and a female part. For example, the male parts 121 may be arranged on the substrate 12 in a form of hollow cylinders 121, arranged on the back side 12b and protruding out of the substrate 12 11, as schematically shown in Fig. 1D, 1E. The female parts of the snap fasteners may be arranged as the outlet tubes 22 of the manifold 20. Each outlet tube 22 and each hollow cylinder 121 may be complementary shaped to form the interlocking pair. Thus, in order to fasten the interlocking parts, the hollow cylinders 121 and the outlet tubes 22, the deformable layer 30 may comprise the apertures through which the hollow cylinders 121 are provided through the deformable layer, so as to join the substrate 12 and the manifold 20, with the deformable layer 30 sandwiched therebetween. Each hollow cylinder 121 comprise hollow body. Thus, upon fastening of the interlocking parts, the hollow cylinder 121 may form a jacket pipe of the outlet tube 22 enabling the conductive medium to flow through the outlet tube 22 undisturbed. The deformable layer 30 may have openings through which the hollow cylinders are transferred to be joined with the outlet tubes 22 of the system 2 for supplying a conductive medium.

Furthermore, the substrate 12 may be connected with the manifold 20 via other fastening means 23, e.g. fastening clips 23 shown in Fig. 5. In this configuration, the deformable layer 30 (not shown in Fig. 5 for clarity) may be also provided with the openings to transfer the connecting elements, e.g. clips of fastening means 23 through the material of the deformable layer.

The outlet tubes 22 of each electrode unit 10 are provided through the substrate 12 and, in the same manner, through the deformable layer 30. Thus, the conductive medium is supplied to the working area undisturbed. The electrode units 10, attached to the deformable layer 30, are spaced from each other so that, upon deformation of the deformable layer 30, the electrode units 10 may move relative to each other so as to fit the working area of the surface electrode to the user's body shape. The smaller the dimensions of each electrode unit, the greater fitting may be achieved. Thus, for the embodiment in which one electrode unit 10 comprises only one component electrode 11, and the surface electrode comprises a plurality of the electrode units 10, evenly distributed on the deformable layer 30, the most exact fitting may be achieved.

However, it is preferred to provide at least four, or more preferably at least nine component electrodes 11 in a single electrode unit 10. This provides better stability of the electrode unit 10 as each component electrode 11 provides one point of contact with the skin.

The component electrodes 11 may constitute an integral part of the substrate 12, with both the substrate 12 and the component electrodes 11 made of one piece of material, e.g. stainless steel. For example, the substrate 12 and the component electrodes 11 may be 3D printed as one integral part.

The component electrodes are made of non-expandable material, thus, the clearance sizes are maintained unchanged during the electrode lifespan; the component electrodes do not comprise disposable endings, thus, no wastes are generated as a result of electrostimulation. The component electrodes preferably are entirely made of metal or metal alloys, such as stainless steel, gold, or silver optionally coated with silver chloride. Alternatively, electroconductive polymers may be used, e.g. ethylene propylene diene monomer (EPDM) matrix mixed with carbon or stainless steel fibers.

Alternatively, the substrate 12 and the component electrodes 11 may be made of individual parts, which are joined together. For example, the substrate 12 may be made of a rigid material, such as rigid plastic or metal plate, thus ensuring the rigidity of a single electrode unit 10 and at the same time deformability of the whole surface electrode, wherein the latter is ensured by the deformable layer 30. The application of rigid substrates 12 provides good stability of the component electrodes 11 within a single electrode unit 10.

Nonetheless, the substrate 12 may be made of flexible material, such as flexible plastic plates. The flexibility of the substrate 12 may provide a better fit of the electrode to the body shape.

## Claims

1. A surface electrode for use on a skin, the surface electrode comprising:
- electrode units (10) connectable to a power supply, each electrode unit (10) comprising:
- a substrate (12) having a front side (12a) and a back side (12b), and
- component electrodes (11, 11.1, 11.2) disposed on the front side (12a) of the substrate (12) and spaced apart from each other such that a free space volume (111) exists between the component electrodes (11, 11.1, 11.2), each component electrode (11) having a proximal end (11b) connected with the substrate (12), a distal end (11a, 11.1a, 11.2a) configured for contacting the skin, and a middle portion (11c) between the proximal end (11b) and the distal end (11a, 11.1a, 11.2a),
wherein the component electrodes (11) are made of a non-expandable material
**characterized in that**
within each of the component electrodes (11, 11.1, 11.2) a thickness (T) of the distal end (11a, 11.1a, 11.2a) is greater than a thickness (t) of the middle portion (11c).

2. The surface electrode according to claim 1 wherein the distal end (11a, 11.1a, 11.2a) of each of the component electrodes (11, 11.1, 11.2) has a thickness (T) which is at least 2 times greater than the thickness (t) of the middle portion (11c) of this component electrode (11).

3. The surface electrode according to claim 1 or 2 wherein the component electrodes (11, 11.1) have the distal ends (11a) of a shape independently selected from the group consisting of: a sphere, a hemisphere, a roll, a cylinder, a stick-like shape, a pin-like shape, a spike-like shape, and a pyramidal shape.

4. The surface electrode according to claim 1 or 2 wherein the component electrodes (11, 11.2) have the distal ends (11.2a) of a mushroom-like shape.

5. The surface electrode according to any of the preceding claims wherein all the component electrodes (11, 11.1, 11.2) within each of the electrode units (10) have the distal ends (11a, 11.1a, 11.2a) of the same shape.

6. The surface electrode according to any of the preceding claims wherein the middle portions (11c) of the component electrodes (11, 11.1, 11.2) have a shape of a cylinder of a radius (r) defining half of the thickness (t) of the middle portion (11c).

7. The surface electrode according to any of the preceding claims wherein the distal ends (11a, 11.1a, 11.2a) of the component electrodes (11, 11.1, 11.2) have convex surfaces for contacting the skin, wherein said convex surfaces are defined by a radius of curvature (C) of said convex surfaces.

8. The surface electrode according to claims 3, 6, and 7 wherein the distal ends (11a, 11.1a) of the component electrodes (11, 11.1) have a shape of a sphere with a radius (R) defining half of the thickness (T) of the distal end (11a, 11.1a), and wherein said radius (R) is equal to the radius of curvature (C) of the surface of the distal end (11a, 11.1a), said radius of curvature (C) being at least 2 times greater than the radius (r) of the middle portion (11c), within each of the component electrodes (11, 11.1).

9. The surface electrode according to claim 4, 6, and 7 wherein the distal ends (11.2a) of the component electrodes (11.2) have a mushroom-like shape with a radius (R) defining half of the thickness (T) of the distal end (11.2a), and wherein said radius (R) is smaller than the radius of curvature (C) of the surface of the distal end (11.2a), said radius of curvature (C) being at least 2 times greater than the radius (r) of the middle portion (11c), within each of the component electrodes (11.2).

10. The surface electrode according to any of the preceding claims wherein each of the electrode units (10) comprises the component electrodes (11, 11.1, 11.2) spaced apart such that a clearance of 1 to 5 mm exists between two adjacent distal ends (11a, 11.1a, 11.2a) so that hair can be introduced and removed from the free space volume (111).

11. The surface electrode according to any of the preceding claims further comprising a system (2) for supplying a conductive medium to the free space volume (111), the system comprising a manifold (20) arranged on the back side (12b) of the substrate (12), the manifold (20) comprising ducts (21) ended with outlet tubes (22) arranged in the substrate (12) from the back side (12b) to the front side (12a) for supplying the conductive medium to the component electrodes (11).

12. The surface electrode according to claim 11 wherein the electrode units (10) are arranged on a deformable layer (30) that is disposed between the manifold (20) and the substrate (12) facing the back side (12b) of the substrate, wherein the deformable layer (30) has openings for disposing the outlet tubes (22) from one side of the deformable layer (30) to the other side, and wherein the manifold (20) is connected with the substrate (12) by fastening means (23, 121).

13. The surface electrode according to any of the preceding claims comprising at least two electrode units (10) and wherein one electrode unit (10) comprises at least four component electrodes (11, 11.1, 11.2).

14. The surface electrode according to any of the preceding claims wherein the component electrodes (11, 11.1, 11.2) are made of electroconductive polymer, metal or metal alloy.

15. The surface electrode according to claim 1 wherein the component electrodes (11) comprise:
- the distal ends (11a) of a distal end thickness (T) having a shape selected from the group consisting of sphere, hemisphere, roll, cylinder, stick-like, pin-like, spike-like, and pyramidal shape,
- the middle portions (11c) of a middle portion thickness (t), and
- the proximal ends (11b) having a shape selected from the group consisting of roll, cylinder, stick-like, pin-like, spike-like, and pyramidal shape.

## Patentansprüche

1. Oberflächenelektrode zur Verwendung auf einer Haut, wobei die Oberflächenelektrode Folgendes umfasst:
- Elektrodeneinheiten (10), die mit einer Stromversorgung verbindbar sind, wobei jede Elektrodeneinheit (10) Folgendes umfasst:
- ein Substrat (12) mit einer Vorderseite (12a) und einer Rückseite (12b), und
- Komponentenelektroden (11, 11.1, 11.2), die auf der Vorderseite (12a) des Substrats (12) vorgesehen und voneinander beabstandet sind, sodass ein Freiraumvolumen (111) zwischen den Komponentenelektroden (11, 11.1, 11.2) existiert, wobei jede Komponentenelektrode (11) ein proximales Ende (11b), das mit dem Substrat (12) verbunden ist, ein distales Ende (11a, 11.1a, 11.2a), das zum Kontaktieren der Haut konfiguriert ist, und einen mittleren Abschnitt (11c) zwischen dem proximalen Ende (11b) und dem distalen Ende (11a, 11.1a, 11.2a) aufweist,
wobei die Komponentenelektroden (11) aus einem nicht erweiterbaren Material bestehen,
**dadurch gekennzeichnet, dass**
innerhalb jeder der Komponentenelektroden (11, 11.1, 11.2) eine Dicke (T) des distalen Endes (11a, 11.1a, 11.2a) größer als eine Dicke (t) des mittleren Abschnittes (11c) ist.

2. Oberflächenelektrode nach Anspruch 1, wobei das distale Ende (11a, 11.1a, 11.2a) von jeder der Komponentenelektroden (11, 11.1, 11.2) eine Dicke (T) aufweist, die zumindest 2-mal größer als die Dicke (t) des mittleren Abschnittes (11c) dieser Komponentenelektrode (11) ist.

3. Oberflächenelektrode nach Anspruch 1 oder 2, wobei die distalen Enden (11a) der Komponentenelektroden (11, 11.1) eine Form aufweisen, die unabhängig ausgewählt ist aus der Gruppe bestehend aus: einer Kugel, einer Halbkugel, einer Rolle, einem Zylinder, einer stabähnlichen Form, einer stiftähnlichen Form, einer spitzenähnlichen Form und einer Pyramidenform.

4. Oberflächenelektrode nach Anspruch 1 oder 2, wobei die distalen Enden (11.2a) der Komponentenelektroden (11, 11.2) eine pilzähnliche Form aufweisen.

5. Oberflächenelektrode nach einem der vorhergehenden Ansprüche, wobei alle der Komponentenelektroden (11, 11.1, 11.2) innerhalb jeder der Elektrodeneinheiten (10) die distalen Enden (11a, 11.1a, 11.2a) der gleichen Form aufweisen.

6. Oberflächenelektrode nach einem der vorhergehenden Ansprüche, wobei die mittleren Abschnitte (11c) der Komponentenelektroden (11, 11.1, 11.2) eine Form eines Zylinders mit einem Radius (r) aufweisen, der die Hälfte der Dicke (t) des mittleren Abschnittes (11c) definiert.

7. Oberflächenelektrode nach einem der vorhergehenden Ansprüche, wobei die distalen Enden (11a, 11.1a, 11.2a) der Komponentenelektroden (11, 11.1, 11.2) konvexe Oberflächen zum Kontaktieren der Haut aufweisen, wobei die konvexen Oberflächen durch einen Krümmungsradius (C) der konvexen Oberflächen definiert sind.

8. Oberflächenelektrode nach Anspruch 3, 6 und 7, wobei die distalen Enden (11a, 11.1a) der Komponentenelektroden (11, 11.1) eine Form einer Kugel mit einem Radius (R) aufweisen, der die Hälfte der Dicke (T) des distalen Endes (11a, 11.1a) definiert und wobei der Radius (R) gleich dem Krümmungsradius (C) der Oberfläche des distalen Endes (11a, 11.1a) ist, wobei der Krümmungsradius (C) zumindest 2-mal größer als der Radius (r) des mittleren Abschnittes (11c) innerhalb jeder der Komponentenelektroden (11, 11.1) ist.

9. Oberflächenelektrode nach Anspruch 4, 6 und 7, wobei die distalen Enden (11.2a) der Komponentenelektroden (11.2) eine pilzähnliche Form mit einem Radius (R) aufweisen, der die Hälfte der Dicke (T) des distalen Endes (11.2a) definiert, und wobei der Radius (R) kleiner als der Krümmungsradius (C) der Oberfläche des distalen Endes (11.2a) ist, wobei der Krümmungsradius (C) zumindest 2-mal größer als der Radius (r) des mittleren Abschnittes (11c) innerhalb jeder der Komponentenelektroden (11.2) ist.

10. Oberflächenelektrode nach einem der vorhergehenden Ansprüche, wobei jede der Elektrodeneinheiten (10) die Komponentenelektroden (11, 11.1, 11.2) umfasst, die voneinander beabstandet sind, sodass ein Abstand von 1 bis 5 mm zwischen zwei benachbarten distalen Enden (11a, 11.1a, 11.2a) existiert, sodass Haar in das Freiraumvolumen (111) eingeführt und daraus entfernt werden kann.

11. Oberflächenelektrode nach einem der vorhergehenden Ansprüche, ferner umfassend ein System (2) zum Versorgen des Freiraumvolumens (111) mit einem leitenden Medium, wobei das System einen Verteiler (20) umfasst, der auf der Rückseite (12b) des Substrats (12) angeordnet ist, wobei der Verteiler (20) Kanäle (21) umfasst, die mit Auslassrohren (22) enden, die in dem Substrat (12) von der Rückseite (12b) zu der Vorderseite (12a) angeordnet sind, um die Komponentenelektroden (11) mit dem leitenden Medium zu versorgen.

12. Oberflächenelektrode nach Anspruch 11, wobei die Elektrodeneinheiten (10) auf einer verformbaren Schicht (30) angeordnet sind, die zwischen dem Verteiler (20) und dem Substrat (12) der Rückseite (12b) des Substrats zugewandt vorgesehen ist, wobei die verformbare Schicht (30) Öffnungen zum Vorsehen der Auslassrohre (22) von einer Seite der verformbaren Schicht (30) zu der anderen Seite aufweist, und wobei der Verteiler (20) mit dem Substrat (12) durch Befestigungsmittel (23, 121) verbunden ist.

13. Oberflächenelektrode nach einem der vorhergehenden Ansprüche, umfassend zumindest zwei Elektrodeneinheiten (10) und wobei eine Elektrodeneinheit (10) zumindest vier Komponentenelektroden (11, 11.1, 11.2) umfasst.

14. Oberflächenelektrode nach einem der vorhergehenden Ansprüche, wobei die Komponentenelektroden (11, 11.1, 11.2) aus elektrisch leitendem Polymer, Metall oder Metalllegierung bestehen.

15. Oberflächenelektrode nach Anspruch 1, wobei die Komponentenelektroden (11) Folgendes umfassen:
- die distalen Enden (11a) einer distalen Enddicke (T) mit einer Form, die ausgewählt ist aus der Gruppe bestehend aus Kugel-, Halbkugel-, Rollen-, Zylinder-, stabähnlicher, stiftähnlicher, spitzenähnlicher und Pyramidenform,
- die mittleren Abschnitte (11c) einer mittleren Abschnittsdicke (t), und
- die proximalen Enden (11b) mit einer Form, die ausgewählt ist aus der Gruppe bestehend aus Rollen-, Zylinder-, stabähnlicher, stiftähnlicher, spitzenähnlicher und Pyramidenform.

## Revendications

1. Electrode de surface destinée à être utilisée sur une peau, l'électrode de surface comprenant :
- des unités d'électrode (10) pouvant être connectées à une alimentation électrique, chaque unité d'électrode (10) comprenant :
- un substrat (12) comportant une face avant (12a) et une face arrière (12b), et
- des électrodes à composants (11, 11.1, 11.2) disposées sur la face avant (12a) du substrat (12) et espacées les unes des autres de sorte qu'un volume d'espace libre (111) existe entre les électrodes à composants (11, 11.1, 11.2), chaque électrode à composants (11) comportant une extrémité proximale (11b) connectée au substrat (12), une extrémité distale (11a, 11.1a, 11.2a) conçue pour entrer en contact avec la peau, et une partie médiane (1 1c) entre l'extrémité proximale (11b) et l'extrémité distale (11a, 11.1a, 11.2a),
lesdites électrodes à composants (11) étant constituées d'un matériau non extensible
**caractérisée en ce que**
à l'intérieur de chacune des électrodes à composants (11, 11.1, 11.2), l'épaisseur (T) de l'extrémité distale (11a, 11.1a, 11,2a) est supérieure à l'épaisseur (t) de la partie médiane (11c).

2. Electrode de surface selon la revendication 1, ladite extrémité distale (11a, 11.1a, 11.2a) de chacune des électrodes à composants (11, 11.1, 11.2) comportant une épaisseur (T) qui est au moins 2 fois supérieure à l'épaisseur (t) de la partie médiane (11c) de cette électrode à composants (11).

3. Electrode de surface selon la revendication 1 ou 2, lesdites électrodes à composants (11, 11.1) comportant des extrémités distales (11a) d'une forme choisie indépendamment dans le groupe constitué par : une sphère, un hémisphère, un rouleau, un cylindre, une forme de type bâton, une forme de type broche, une forme de type pointe et une forme pyramidale.

4. Electrode de surface selon la revendication 1 ou 2, lesdites électrodes à composants (11, 11.2) comportant les extrémités distales (11.2a) d'une forme de type champignon.

5. Electrode de surface selon l'une quelconque des revendications précédentes, toutes les électrodes à composants (11, 11.1, 11.2) à l'intérieur de chacune des unités d'électrode (10) comportant des extrémités distales (11a, 11.1a, 11.2a) de la même forme.

6. Electrode de surface selon l'une quelconque des revendications précédentes, lesdites parties médianes (1 1c) des électrodes à composants (11, 11.1, 11.2) comportant une forme d'un cylindre d'un rayon (r) définissant la moitié de l'épaisseur (t) de la partie médiane (11c).

7. Electrode de surface selon l'une quelconque des revendications précédentes, lesdites extrémités distales (11a, 11.1a, 11.2a) des électrodes à composants (11, 11.1, 11.2) comportant des surfaces convexes destinées à entrer en contact avec la peau, lesdites surfaces convexes étant définies par un rayon de courbure (C) desdites surfaces convexes.

8. Electrode de surface selon les revendications 3, 6 et 7, lesdites extrémités distales (11a, 11.1a) des électrodes à composants (11, 11.1) comportant une forme d'une sphère avec un rayon (R) définissant la moitié de l'épaisseur (T) de l'extrémité distale (11a, 11.1a), et ledit rayon (R) étant égal au rayon de courbure (C) de la surface de l'extrémité distale (11a, 11.1a), ledit rayon de courbure (C) étant au moins 2 fois supérieur au rayon (r) de la partie médiane (11c), à l'intérieur de chacune des électrodes à composants (11, 11.1).

9. Electrode de surface selon la revendication 4, 6 et 7, lesdites extrémités distales (11.2a) des électrodes à composants (11.2) comportant une forme de type champignon avec un rayon (R) définissant la moitié de l'épaisseur (T) de l'extrémité distale (11.2a), et ledit rayon (R) étant inférieur au rayon de courbure (C) de la surface de l'extrémité distale (11.2a), ledit rayon de courbure (C) étant au moins 2 fois supérieur au rayon (r) de la partie médiane (11c), à l'intérieur de chacune des électrodes à composants (11.2).

10. Electrode de surface selon l'une quelconque des revendications précédentes, chacune des unités d'électrode (10) comprenant les électrodes à composants (11, 11.1, 11.2) espacées de sorte qu'il existe un jeu de 1 à 5 mm entre deux extrémités distales adjacentes (11a, 11.1a, 11.2a) afin que des poils puissent être introduits et retirés du volume d'espace libre (111).

11. Electrode de surface selon l'une quelconque des revendications précédentes, comprenant en outre un système (2) destiné à fournir un milieu conducteur au volume d'espace libre (111), le système comprenant un collecteur (20) disposé sur la face arrière (12b) du substrat (12), le collecteur (20) comprenant des conduits (21) terminés par des tubes de sortie (22) disposés dans le substrat (12) de la face arrière (12b) à la face avant (12a) destinés à fournir le milieu conducteur aux électrodes à composants (11).

12. Electrode de surface selon la revendication 11, lesdites unités d'électrode (10) étant disposées sur une couche déformable (30) qui est disposée entre le collecteur (20) et le substrat (12) faisant face à la face arrière (12b) du substrat, ladite couche déformable (30) comportant des ouvertures destinées à disposer les tubes de sortie (22) d'un côté de la couche déformable (30) à l'autre côté, et ledit collecteur (20) étant relié au substrat (12) par des moyens de fixation (23, 121).

13. Electrode de surface selon l'une quelconque des revendications précédentes, comprenant au moins deux unités d'électrode (10) et une unité d'électrode (10) comprenant au moins quatre électrodes à composants (11, 11.1, 11.2).

14. Electrode de surface selon l'une quelconque des revendications précédentes, lesdites électrodes à composants (11, 11.1, 11.2) étant constituées d'un polymère, d'un métal ou d'un alliage métallique électroconducteur.

15. Electrode de surface selon la revendication 1, lesdites électrodes à composants (11) comprenant :
- les extrémités distales (11a) d'une épaisseur d'extrémité distale (T) comportant une forme choisie dans le groupe constitué par une forme en sphère, en hémisphère, en rouleau, en cylindre, de type bâton, de type broche, de type pointe et pyramidale,
- les parties médianes (11c) d'une épaisseur de partie médiane (t), et
- les extrémités proximales (11b) comportant une forme choisie dans le groupe constitué par une forme en rouleau, en cylindre, de type bâton, de type broche, de type pointe et pyramidale.
